# EUROPEAN PATENT APPLICATION

(11) **EP 3 581 117 A1**
(43) Date of publication of application: **18.12.2019**
(21) Application number: 19185244.1
(22) Date of filing: 12.03.2014
(51) Int. Cl.: A61B 17/06

(54) **BLUNT NEEDLE FOR DELIVERY OF DERMAL FILLER THREADS**

(30) Priority: 12.03.2013 US 201361778066 P
(62) Divisional of application: 14780054.4
(71) Applicant: Allergan Holdings France S.A.S., 92400 Courbevoie (FR)
(72) Inventor: SOLISH, Nowell, Menlo Park, California 94025 (US); HORNE, Kenneth, Menlo Park, California 94025 (US); GIPSON, Tony, Menlo Park, California 94025 (US)
(74) Representative: Hoffmann Eitle

(57) **Abstract**

Provided herewith is a needle for delivering dermal filler thread to a wrinkle in a patient. The needle can also be used to deliver the thread to a patient for the purposes of facial contouring. The needle comprises a coupler for attaching the thread to the needle and a blunt distal end to ease delivery through the skin.

## Description

### Field

This relates generally to delivery devices for dermal filler threads. The delivery device is useful for delivering a thread to a patient, for example a facial wrinkle.

### State of the Art

Dermal fillers have become prevalent in the field of aesthetic intervention. When dermal fillers were first studied in the 1980's, animal derived collagen fillers were most popular. However, due to skin allergies, dermal fillers of hyaluronic acid have become preferred over collagen due to fewer allergic reactions and better pliability. To date, dermal fillers approved for use in human patients to fill wrinkles consist mainly of gel compositions. Examples of these gel compositions include those made with hyaluronic acid, such as Restylane® and Juvederm®.

As gels can be difficult to deliver in a targeted manner to wrinkles in the face, more structural forms, such as a threads, are currently being investigated. As described in WO 2010/028025, to fill a wrinkle with a thread, the thread is attached to a needle at its proximal end. The distal end of the needle is then inserted through the skin surface of the subject into the dermis (or other layer) adjacent to or within the wrinkle. The needle then traverses the dermis of the subject. The needle exits the skin and by pulling the needle distally, the thread is deposited into the wrinkle. Heretofore, an effective means of attaching the thread to the needle for depositing a thread into a wrinkle has not been described.

### Summary

Provided is a device for delivering a dermal filler thread to a patient.

In one embodiment is provided a needle for delivering a dermal filler comprising a tubular body having a proximal portion and a distal portion, a coupler in its proximal portion for mechanically attaching a dermal filler to the needle, and a blunt distal end in its distal portion.

In another embodiment is provided a kit comprising at least one needle for delivering a dermal filler comprising a tubular body having a proximal portion and a distal portion, a coupler in its proximal portion for mechanically attaching a dermal filler to the needle, and a blunt distal end in its distal portion The needle of the kit is coupled to a thread which is biocompatible and compressible. In one embodiment, the thread is comprised of hyaluronic acid, salt, hydrate or solvate thereof optionally wherein at least a portion of the hyaluronic acid, salt, hydrate or solvate thereof is cross-linked.

Further embodiments are described throughout.

### Detailed Description of the Drawings

The following detailed description is best understood when read in conjunction with the drawings. It should be noted that the various features of the drawings may not be to-scale. On the contrary, dimensions of certain features are arbitrarily expanded or reduced for clarity. Included in the drawings are the following figures:
Fig. 1 is a schematic illustration of one embodiment of a needle having a coupler comprising struts.
Fig. 2 is a schematic illustration of a needle having a coupler comprising a funnel.
Fig. 3 is an exploded view of 1A from Figure 1. The close-up shows a coupler having cleats in the shape of tabs.
Fig. 4 is an alternative exploded view of 1A from Figure 1. The close-up shows a coupler having cleats in the shape of teeth.
Fig. 5 is a side view of a coupler having three pairs of struts where the pairs are symmetrically placed along the longitudinal axis of the needle. This embodiment is sometimes referred to as "mirrored struts".
Fig. 6 is a side view of a coupler having three pairs of struts where the pairs are staggered along the longitudinal axis of the needle.
Fig. 7 is a side view of a coupler having cleats in the shape of teeth.
Fig. 8 is a perspective view of a coupler having tabs and struts.
Fig. 9 is a perspective view of a coupler having slits and no struts.
Fig. 10 is a perspective view of a coupler having struts and teeth-shaped cleats.
Fig. 11 is a perspective view of a coupler having three slits with staggered struts.
Fig. 12 is a side view of a coupler having v-shaped strut and teeth-shaped cleats.
Fig. 13 is a side view of a coupler having a u-shaped strut and teeth-shaped cleats.
Fig. 14 is a side view of a coupler having u-shaped struts and teeth-shaped cleats.
Fig. 15 is a perspective view of a blunt distal end.
Fig. 16 is a perspective view of a thread attached or coupled to an exemplary needle described herein.
Fig. 17 is a schematic illustration of one embodiment of a needle having a hypotube coupler.
Fig. 18 is an exploded view of 19A from Fig. 17. The close-up shows a coupler having cleats in the shape of tabs.
Fig. 19 is a face view of a hypotube coupler.
Fig. 20 is a photograph and a schematic of the side view of an angled hypotube coupler attached to a needle.
Fig. 21 is a photograph and a schematic of the side view of a flush hypotube coupler attached to a needle.
Fig. 22 is a photograph and a schematic of the side view of a hypotube coupler having eight crimp points.
Fig. 23 is a photograph and a schematic of the side view of a hypotube coupler having four crimp points.
Fig. 24 is a photograph of the side view of a needle having a hypotube coupler with four crimp points attached to a thread.
Fig. 25 is a schematic of a needle having a detachable coupler with three threads of different diameters having a coupler attached.
Fig. 26A is a schematic of a needle having a detachable coupler inserted through the dermis.
Fig. 26B is a schematic of a needle having a detachable coupler inserted through the dermis with a thread attached.
Fig. 27A is a schematic of a straight sheath.
Fig. 27B is a schematic of a sheath with a pull tab.
Fig. 27C is a schematic of a sheath with a pull tab and an insertion preventer.
Fig. 28A, Fig. 28B, and Fig. 28C are schematics of a sheath with a pull tab and an insertion preventer as it is inserted into and pushed through the dermis.
Fig. 29 is a schematic of a sheath, needle and thread further with a needle grabber.
Fig. 30A and Fig. 30B are schematics of a self-buckling sheath.
Fig. 31 is a schematic of a self-buckling sheath after insertion of the needle and thread into the dermis.
Fig. 32A and Fig. 32B are schematics of a self-buckling sheath after insertion of the needle and thread into the dermis.

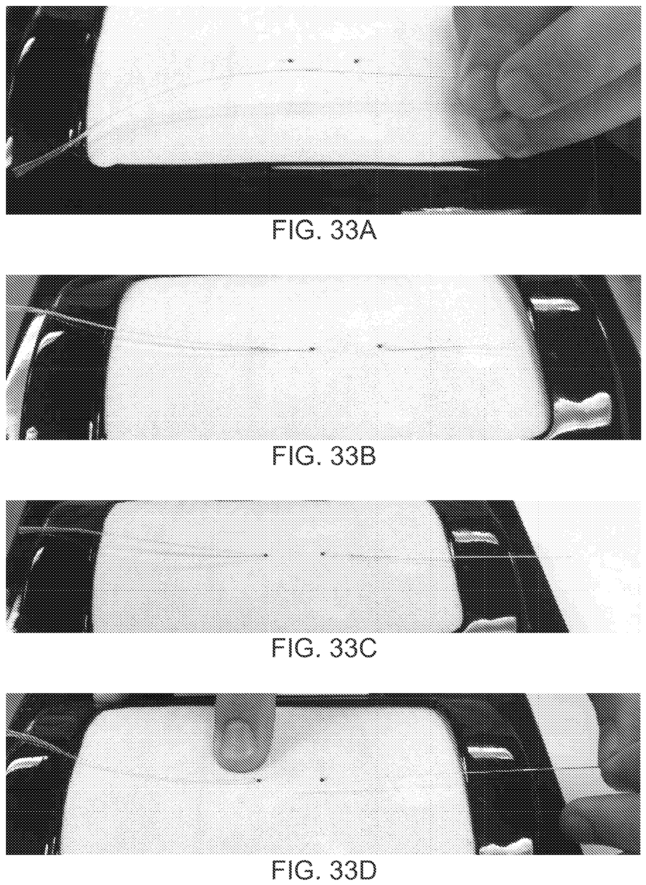

Fig. 33A shows a sheathed needle and thread.
Fig. 33B shows a sheathed needle and thread inserted into puppet skin.
Fig. 33C shows a sheathed needle pulled through puppet skin, where the sheath is held at the insertion site, effectively removing sheath by entirely passive means.
Fig. 33D shows the sheath completely removed from the needle and thread.

### Detailed Description

### Device

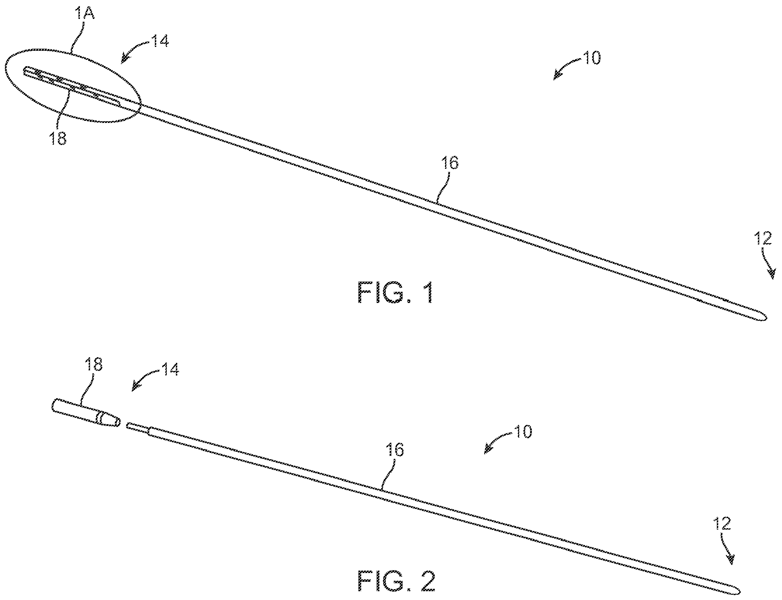

Provided in Fig. 1 is one embodiment of a needle 10 having a distal portion or end 12, a proximal portion or end 14, and a tubular body 16. In some instances, the tubular body 16 may either have a lumen or be solid depending on the stiffness desired. In all instances, the tubular body will have sufficient stiffness to inserted into and through dermis, epidermis, and/or subcutaneous tissue.

The proximal portion or end 14 comprises a coupler 18 as shown in various embodiments in Fig. 1, Fig. 2, Fig. 3, Fig. 4, Fig. 5, Fig. 6, Fig. 7, Fig. 8, Fig. 9, and Fig. 11. As used herein, the terms "portion" and "end" are used interchangeably. The coupler 18 is a means for mechanically attached a thread to the needle 10 for delivery to any layer of skin. The coupler at its proximal end 20 (as shown in the figures just mentioned) has a inner diameter that is substantially the same as the outer diameter of the thread that is being attached. In one embodiment, the coupler 18 is continuous with the tubular body of the needle 10, meaning that the distal end of the needle 10 is manipulated (e.g., laser cut or crimped) to serve as the coupler 18. In another embodiment, the coupler 18 is a separate piece from the needle 10 and is affixed thereto by any number of means.

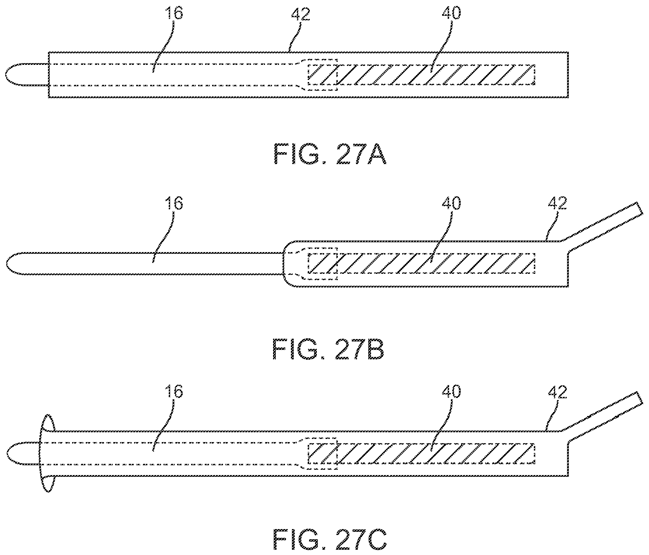

In some embodiments, the needle 10 further comprises a sheath (Fig. 27A, Fig. 27B, and Fig. 27C). The sheath fits over the coupler 18 once the thread 10 is attached to provide additional structural support to the coupler 18. The sheath may be thin-walled heat shrink material, such as polyethylene teraphthalate (PET) or polytetrafluroethylene (PTFE).

In one embodiment, the coupler 18 is designed such that it can be expanded to a greater inner diameter and thus allow a portion of the thread to be placed into the coupler. The thread is then inserted through the length or a substantial portion of the length of the coupler. Once the thread is placed into the coupler, the expanded coupler is then crimped or closed to a diameter that fits the thread. In some instances, it is an inner diameter more similar to its unexpanded inner diameter or even smaller. The coupler 18 is crimped to an outer diameter to allow for ease of delivery through the skin but still allows the thread to maintain its structural integrity. Once this crimping occurs, the thread is mechanically attached to the coupler and thus, the needle. In one embodiment, the expansion is about 120% (i.e., from .010" to .022" inner diameter (ID)) and crimping back down to 110-150% (0.011"-0.015" ID).

Due to the design of the coupler, the thread is not easily detached from the coupler/needle during delivery to aid in the accurate positioning of the thread.

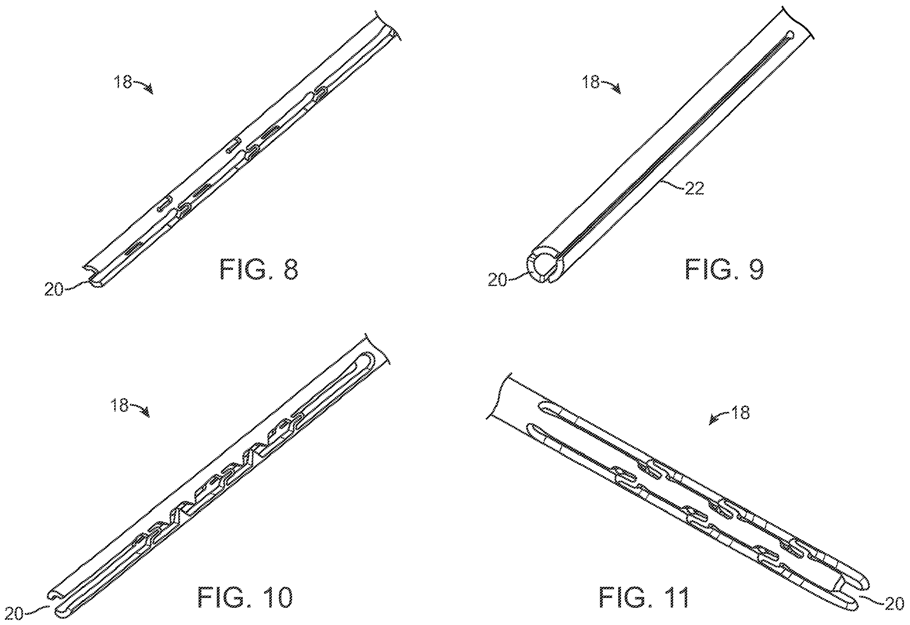

To aid in the ability of the coupler 18 to expand its inner diameter to place the thread, a variety of modifications may be made to the coupler. In one embodiment, one or more slits 22 are made along the longitudinal axis of the coupler. These slits 22 can be made by cutting, laser cutting, chemical etching, or stamping and rolling the coupler. In some embodiments, the coupler will have two or three slits as shown in Fig. 9. In one embodiment, each slit 22 is place about equidistant apart. Depending on whether the coupler 18 is the same or a different piece of material than the needle will determine how long the slit 22 or slits 22 may be. If the coupler is the same piece of material as the needle, the slit(s) may run the entire length of the coupler. Further, the inner edges of the slits 22 may be smooth or serrated.

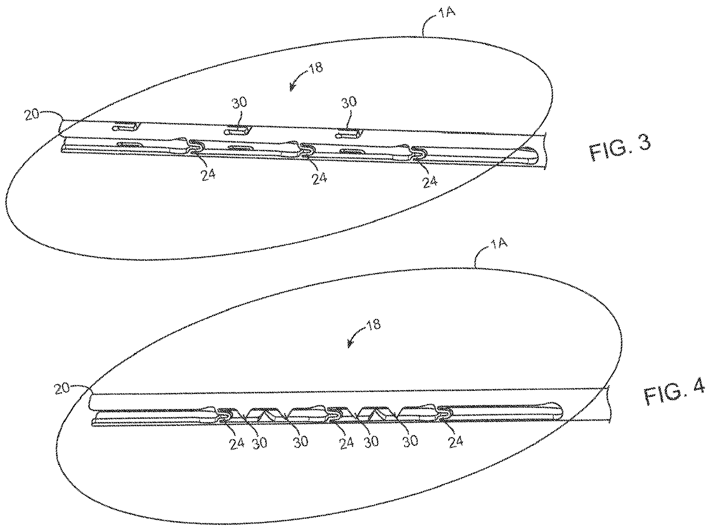

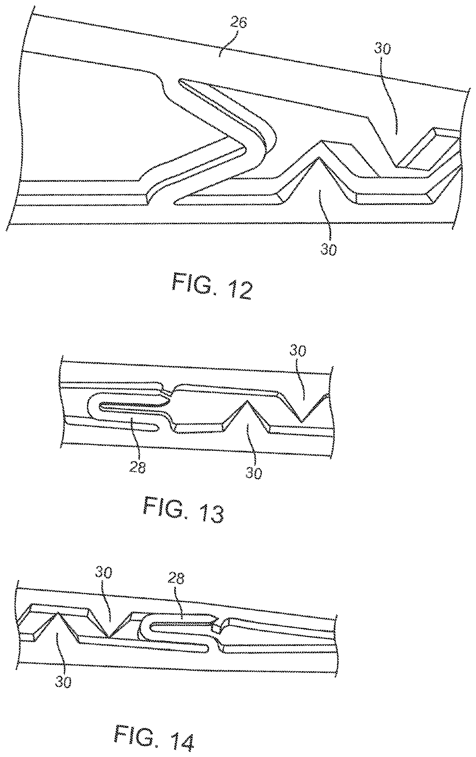

In addition to slits, the coupler may also comprise one or more struts 24. The strut 24 is shown in Fig. 3 and Fig. 4. As shown in Fig. 12, the strut 24 can be v-shaped 26 or as shown in Fig. 13 and Fig. 14, the strut 28 can be u-shaped. Typically, the struts 24 are sized to maintain the integrity of the metal employed. In some embodiments, the ratio of the wall thickness of the coupler 18 to the width of the strut 24 at its widest part is about 1:1. The struts are similar to those used in stent design, however, in vascular stents, the struts allow a stent to be crimped down to a minimal diameter for ease of delivery through a small catheter and then expanded back to their original size. With the struts described herein, the struts allow the coupler to be expanded to receive a diameter thread that is larger than the unexpanded inner diameter. Once the thread is inserted, the struts allow the diameter to be crimped down to a small profile to be threaded through the skin.

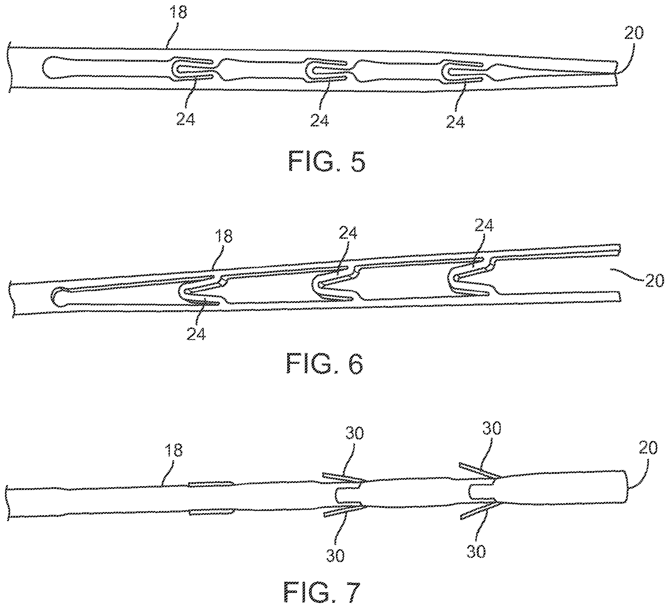

In some embodiments, the slits 22 in the coupler comprise one strut 24 but can comprise up to 6 or 8 struts. As shown in Fig. 5, the coupler 18 can comprise two slits 18 that are cut along the same plane. In those two slits, there are anywhere from one pair to four pair of struts. When the struts are present in even numbers, additional hoop strength is provided to the coupler. In the Fig. 5, there are 3 pairs of struts, although only three of the struts are visible in the side view. The other three struts are in the same location in the other slit, mirroring the slits in view. This is referred to as "mirrored struts." In some embodiments the mirrored struts may be offset. Thus, the needle may comprise one or more pairs of mirrored struts.

Fig. 6 provides a view of one embodiment of the coupler in its expanded position. In this version of the coupler 18, there are two slits 22. In this embodiment, four struts 24 are shown. However, unlike in Fig. 5, the struts are staggered rather than mirrored. This staggered design may be employed regardless of whether there are an even or an odd number of struts.

To further aid in the coupler's ability to engage the thread, the slits 22 may also optionally comprise one or more cleats 30. The cleats 30 may be selected from a variety of shapes, including tab-shaped, like seen in Fig. 3, Fig. 8, and Fig. 11, or teeth-shaped as seen in Fig. 4, Fig. 10, Fig. 12, Fig. 13, and Fig. 14. The cleats can be separately crimped after crimping of the coupler 18 to provide improved retention of the thread by the coupler 18.

In some embodiments, the coupler comprises both struts and cleats to maximize retention of the thread. Various embodiments of a combination of struts 22 and cleats 30 may be seen in Fig. 3, Fig. 4, Fig. 8, Fig. 10, and Fig. 11.

The slits, struts and cleats may all be fashioned via laser cutting or other means.

In addition to the coupler providing the ability to be expanded, the coupler may also serve as a funnel. This is shown in Fig. 2. The thread may be extruded through the funnel and then it can dried or bonded to the inner diameter of the funnel. The funnel then is attached to the proximal end of the needle. Alternatively, the thread could be inserted and adhered with a compatible adhesive into the back end of the funnel. Any number of adhesives may be employed such as cyanoacrylate or other ultraviolet curable adhesives.

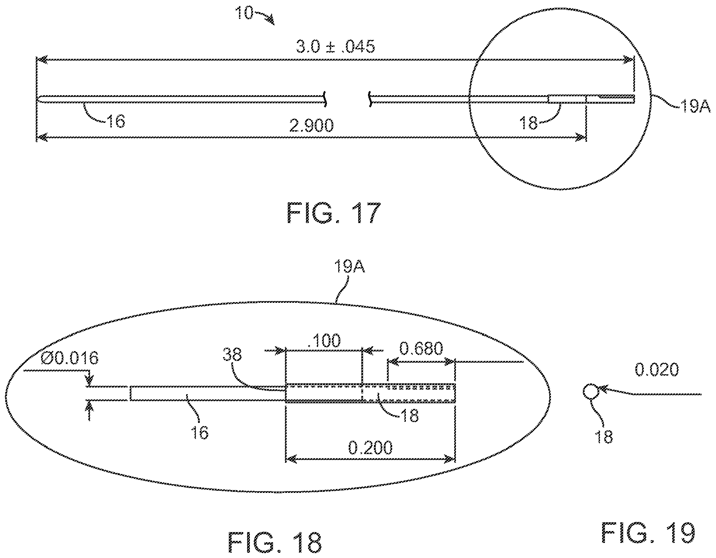

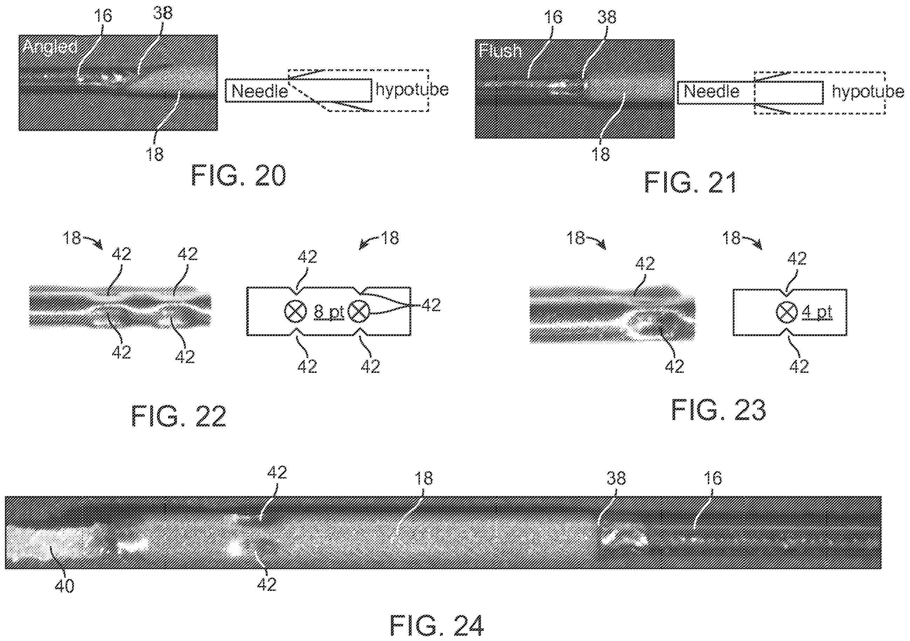

As discussed above, in some embodiments, the coupler 18 is a separate hollow, substantially tubular piece which is attached to the proximal end of the needle 10 as seen in Fig. 17, Fig. 18, Fig. 20, and Fig. 21. This substantially tubular type of coupler is herein referred to as a "hypotube coupler". As seen in Fig 17, the proximal end of the needle is inserted at least partway, and in some embodiments about halfway, into one end of the hypotube coupler and is attached thereto by any number of means, including but not limited to, an adhesive, welding, crimping and/or heat shrink. In some embodiments, the hypotube coupler is laser welded to the proximal end of the needle. In one embodiment, the length of the hypotube coupler is about 0.2 inches (Fig. 18) and has an inner diameter of about 0.02 inches (Fig. 19). As shown in Fig. 20 and Fig. 21, the attachment profile 38 of the hypotube coupler can be angled or flush. In some embodiments, the attachment profile 38 of the hypotube coupler is angled, such that it provides gradual transition with the needle and therefore less drag through the tissue. In some embodiments, the attachment profile 38 of the hypotube coupler is flush.

Once attached to the needle, at least a portion of the hypotube coupler extends beyond the proximal end of the needle for housing the thread. The thread can then be inserted into the remaining portion of the hypotube coupler and affixed thereto. Once the thread is placed into the coupler, the hypotube coupler is then crimped 42 around its circumference to immobilize the thread (see Fig. 22 and Fig. 23). In some instances, the hypotube coupler is crimped 42 at least four times around its circumference in one single plane, thus forming a "four-point crimp", to immobilize the thread (see Fig. 23). Once this crimping occurs, the thread is mechanically attached to the hypotube coupler and thus, the needle. In some embodiments, a series of at least two four-point crimps are used to secure the thread. Fig. 23 shows two four-point crimps forming an "eight-point crimp". The four-point crimp allows the use of a shorter hypotube coupler (e.g., 0.17 inches vs 0.25 inches for the eight-point crimp) with less material. The eight-point crimp demonstrates a higher pull out force (i.e., the force required to dislodge the thread from the hypotube coupler. It is contemplated that the hypotube coupler displays a pull out force of greater than about 0.400 lbf, or greater than 0.600 lbf, or greater than 0.800 lbf, or even greater than 1.00 lbf. In some embodiments, the cumulative drag of the needle 10 having a hypotube coupler 18 and thread 40 attached thereto (e.g., Fig. 24) is less than about 0.20 lbf-in, or less than about 0.10 lbf-in, or less than about 0.075 lbf-in, or less than about 0.060 lbf-in, or from about 0.1 to about 0.01, or from about 0.08 to about 0.04, or from about 0.075 to about 0.045 lbf-in.

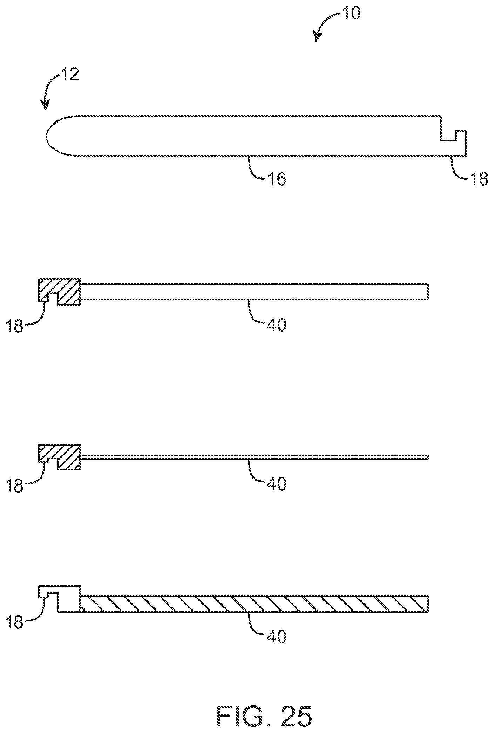

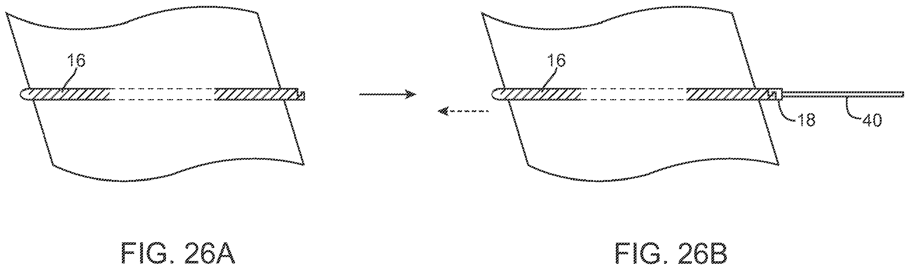

In certain embodiments, the design of the coupler is such that the thread is easily attached and/or detached from the coupler/needle during delivery to aid in the accurate positioning of the thread. Using such a detachable coupler allows the clinician to gauge effect by first inserting the needle 10 into the dermis 50, selecting a thread 40 having the desired thickness and then attaching the thread to the coupler 18 and pulling the thread through the dermis. See, for example, Fig. 25, Fig. 26A, and Fig. 26B.

In all embodiments of the coupler just described, a blunt distal end is employed. Compared to a sharp end that is used in conventional needles, a blunt end cannot pierce the skin like a sharp end. A blunt end, however, has the advantage of being less traumatic to the tissue or cells under the skin. For instance, rather than piercing though a tissue or cell that would otherwise be intact, a blunt end can bypass the intact tissue and penetrate through existing loose tissue or empty space, reducing discomfort during a procedure. Further, a blunt needle can be used to deliver dermal filler threads in more natural tissue planes. Compared to sharp tip needles, which can dissect into and out of tissue planes, the blunt tip will follow the path of least resistance, increasing the likelihood of remaining within the initial tissue plane.

A "blunt distal end" as used herein, refers to a distal end of the coupler having no sharply pointed end. In some aspects, the blunt distal end does not include an angle that is smaller than 90 degrees. Alternatively, the blunt distal end does not include an angle that is smaller than 50 degrees, 60 degree, 70 degrees, 80 degrees, 100 degrees, 110 degrees or 120 degrees. In some embodiments, the blunt distal end does not have an angle pointed end, but rather only includes smooth or round surfaces and edges.

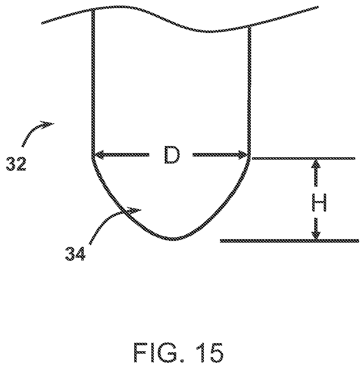

In some embodiments, a blunt distal end includes a tip portion 34 (as illustrated in Fig. 15) that has a height (H) and a substantially round base, which can be of the same size as the body of the coupler. In some embodiments, the height (H) of the tip portion is less than the diameter (D) of the base. In some embodiments, the height (H) of the tip portion is less than about 200%, 180%, 150%, 120% or 80% of the diameter (D) of the base.

Typically, the needle 10, as well as the coupler 18 and the blunt distal end 34 are made of stainless steel. The needle may also optionally include a coating. The coating may serve to enhance the lubricity of the needle, reduce friction, and/or may serve to cover any exposed laser cut edges. The coating may be either hydrophilic or hydrophobic. In some embodiments, the coating is applied by dipping or spraying the coating onto the needle. In some embodiments, the coating is curable at room temperature and may be silicone based, such as a dispersion comprising aminofunctional polydimethylsiloxane copolymer in a mixture of aliphatic and isopropanol solvents. In another embodiment, the coating is a heat-shrinkable material, such as PET or PTFE.

While the dimensions of any component just described, it is contemplated that a 27 gauge needle that is approximately 1" to 4" in length may be employed. The inner diameter of the coupler is about 0.010" and the expanded inner diameter is about 0.021". In some embodiments, the following dimensions are also employed: 1) outer diameter of thread is from about 0.011" to about 0.020"; 2) the length of coupler is about 0.250"; 3) length of the tip portion of the blunt distal end is about 3.0"; 4) other suitable gauge needles include 24-30 gauge.

### Threads

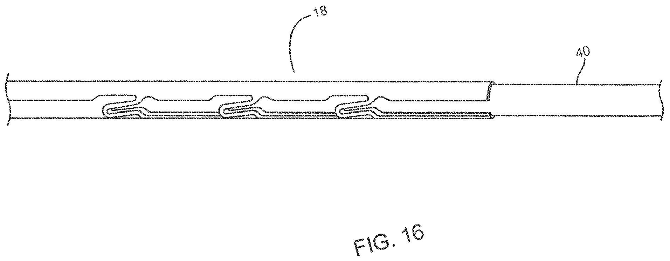

As shown in Fig. 16, a thread 40 is attached to a needle 10 at its proximal end via the coupler 18. Although it is contemplated that any thread can be used, one embodiment is directed to the needle as disclosed herein, with a biocompatible and optionally compressible thread. Biocompatible refers to the fact that a substance will not produce a toxic, injurious, or immunological response in living tissue.

For example, suitable biocompatible threads can comprise epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid polymers, polyvinyl alcohol, 2-hydroxyethyl methacrylate polymers, polymethyl methacrylate, 1,3-bis(carboxyphenoxy)propane polymers, lipids, phosphatidylcholine, triglycerides, polyhydroxybutyrate, polyhydroxyvalerate, poly(ethylene oxide), poly ortho esters, poly (amino acids), polycyanoacrylates, polyphophazenes, polysulfone, polyamine, poly (amido amines), fibrin, graphite, flexible fluoropolymer, isobutyl-based polymers, isopropyl styrene polymers, vinyl pyrrolidone polymers, cellulose acetate dibutyrate polymers, silicone rubber, hyaluronic acid, collagen, chondroitin sulfate, cyclodextrin, alginate, chitosan, carboxy methyl chitosan, heparin, gellan gum, agarose, cellulose, poly (glycerol-sebacate) elastomer, poly(ethylene glycol)-sebacic acid, poly(sebacic acid-co-ricinoleic acid), guar gum, xanthan gum, and combinations and/or derivatives thereof.

In certain embodiments, the threads comprise a thread of hyaluronic acid or salts, hydrates or solvates thereof or a thread of cross linked hyaluronic acid or salts, hydrates or solvates thereof or a combination thereof. Suitable hyaluronic acid threads are known in the art (see, e.g., WO/2010/028025, WO/2011/109130 and WO/2011/109129).

Accordingly, in one aspect, is provided a needle as disclosed herein attached to a thread comprised of hyaluronic acid or salts, hydrates or solvates thereof. In certain embodiments the thread is comprised of cross-linked hyaluronic acid or salts, hydrates or solvates thereof cross linked with butanediol diglycidyl ether (BDDE), divinyl sulfone (DVS) or 1-ethyl-3-(3-dimethylaminopropyl) carbodiimide hydrochloride (EDC). Those of skill in the art will appreciate that many other cross-linking agents may be used to cross-link hyaluronic acid or salts, hydrates or solvates thereof. The above list of cross-linking agents is illustrative rather than comprehensive. In one embodiment, the needle as disclosed herein is attached to a thread comprised of cross-linked hyaluronic acid or salts, hydrates or solvates thereof, wherein the hyaluronic acid has been cross linked with butanediol diglycidyl ether (BDDE).

In some embodiments, the threads comprise a thread of cross-linked hyaluronic acid. For example, in one embodiment, the cross-linked hyaluronic acid is a gel composition comprising at least 5% hyaluronic acid, wherein the hyaluronic acid is substantially cross-linked with at least about 15 mole % of a butanediol diglycidyl ether (BDDE) derivative relative to the repeating disaccharide unit of the hyaluronic acid. In some embodiments, the cross-linked hyaluronic acid further comprises a binder, such as noncross-linked hyaluronic acid.

The threads that include cross-linked hyaluronic acid can be prepared using a composition comprising substantially cross-linked hyaluronic acid, wherein hyaluronic acid is cross-linked with at least about 15 mole % of a butanediol diglycidyl ether (BDDE) derivative relative to the repeating disaccharide unit of the hyaluronic acid. In some embodiments, the composition comprises at least 5% hyaluronic acid before cross-linking, such as 8%, 10% or 12% hyaluronic acid. Further, in some embodiments, the threads include both cross-linked and noncross-linked hyaluronic acid.

Various aspects of the thread manufacturing process (e.g., rinsing, deaeration, extrusion, and drying of precursor gels, as well as the terminal sterilization of the dry threads) can be altered to produce threads having improved physical characteristics, suitable for the present technology. Specifically, threads comprising cross-linked hyaluronic acid can be prepared with significant cross-linking (e.g., at least about 15% BDDE derivative) relative to the repeating disaccharide unit of the hyaluronic acid. Further information about compositions and methods for preparing threads suitable for use in the present technology can be found at United States Patent Application Serial No. 13/649,051, entitled "Threads of cross-linked hyaluronic acid and methods of use thereof," the content of which is incorporated into the present disclosure by reference in its entirety.

### Methods of Delivering the Threads

The needles as disclosed herein, in combination with a biocompatible thread, can be used in aesthetic applications (*e.g*., facial contouring, dermal fillers), surgery (*e.g*., sutures), drug delivery, and the like.

In one aspect, provided is a method of treating a wrinkle in a subject in need thereof. A biocompatible thread is coupled to the proximal aspect of a needle as shown, for example, in Fig. 16. The distal end of the needle 10 or blunt distal end is then inserted through the skin surface of the subject into the dermis adjacent to or within the wrinkle and the dermis of the subject in the base of the wrinkle is traversed with the needle. Once the needle exits the skin surface of the subject, it is pulled distally until it is removed from the skin of the subject such that the thread is pulled into the location previously occupied by the needle. The excess thread is then detached from the needle at the skin surface of the subject. The detachment can be accomplished by cutting or breaking the thread at or below the surface of the skin.

In some embodiments, as the needle has a blunt distal end, a small excision is made at the insertion site before insertion of the needle to facilitate the insertion. In some embodiment, an opening can be cut into the tissue under the skin to guide penetration of the needle through the dermis. The excision and opening can be made with equipments under precise motion control or monitored, and thus can be made with precision. Accordingly, the excision and the opening in the tissue not only facilitate penetration of the needle, but also makes it easier to control the direction and depth of penetration.

In another embodiment, provided is method of providing facial contouring in a subject in need thereof. In this embodiment, the needle attached to a thread is inserted into the dermis at or adjacent to the desired treatment location, e.g., the lips, the nasolabial fold, the tear trough, etc. The needle then applies the thread to the desired area, providing facial contouring. In one embodiment, a thread is applied to various planes of the dermal tissue. In one embodiment, several threads can be placed generally parallel to each other and additional threads places in a generally perpendicular direction with respect to the first set of parallel threads thereby forming a mesh structure whose aggregate effect is to contour a larger defect or more widespread defect such as the tear trough or the infraorbital region of the eye.

Also contemplated are methods of using the needles of the invention attached to biocompatible threads, hyaluronic acid threads for example, in surgery, ophthalmology, wound closure, drug delivery, and the like.

### Further Embodiments

The clinical implementation of the needles attached to biocompatible threads, such as hyaluronic acid threads, as disclosed herein differs from how injectable dermal fillers are currently delivered. For typical injectable fillers, the prefilled syringe is acquired and a sterile needle with a needle cover or cap is attached thereto. Once the needle is attached to the syringe, the needle cover or cap can be removed without the clinician coming in direct contact with the needle. With the present needle and thread, however, it may be the case that the clinician directly handles one or more of the components (i.e. the needle and/or thread) which are being inserted and/or implanted into the patient. Therefore, in some cases it may be desirable to implement a covering or sheath which can protect the entirety or a portion of the needle and thread assembly from exposure and/or contact during the insertion and implantation. Fig. 27A, Fig. 27B, and Fig. 27C show several sheath 42 constructs contemplated herein, each of which could be constructed from material amenable to e-beam sterilization, such as polyethylene terephthalate (PET), and may be colored to prevent inadvertent implantation.

Fig. 27A shows a straight sheath 42 which covers the entire needle 10 and thread 40. The straight sheath is of a diameter slightly larger than the needle and thread such that is readily removed by the clinician, but not so large that is would be displaced during routine handling. Fig. 27B shows a partial sheath 42 which houses the thread 40 without substantially housing the needle 10. In certain embodiments, the sheath can be equipped with a pull tab at the proximal end (See, e.g., Fig. 27B) which the clinician can use to pull off the sheath at the desired time. Such sheaths 42 could be removed either directly prior to or during insertion of the needle 10 and thread 40.

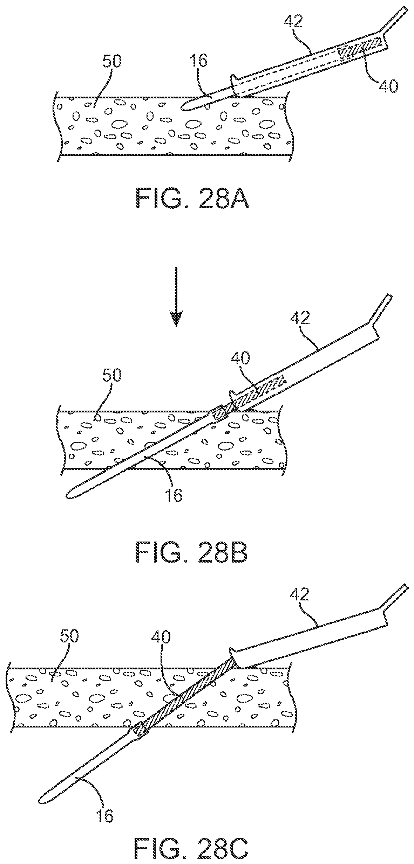

In certain embodiments, the sheath 42 is designed with an expanded distal edge to act as an insertion protector (Fig. 27C, Fig. 28A, Fig. 28B, and Fig. 28C) by abutting the skin as the needle is inserted into the patient while passively removing the sheath from the needle 10 and thread 40.

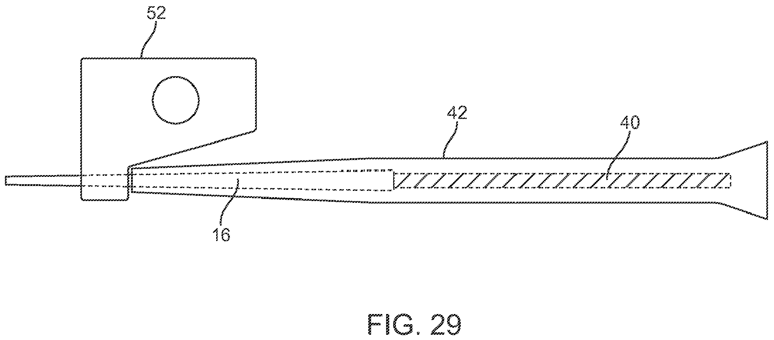

In certain embodiments, the sheath 42, needle 10 and thread 40 further comprise a needle grabber 52 which tightly and securely clamps the needle 10 when grasped by the fingers of the clinician, and then allows the needle to slide freely when tension is released (Fig. 29).

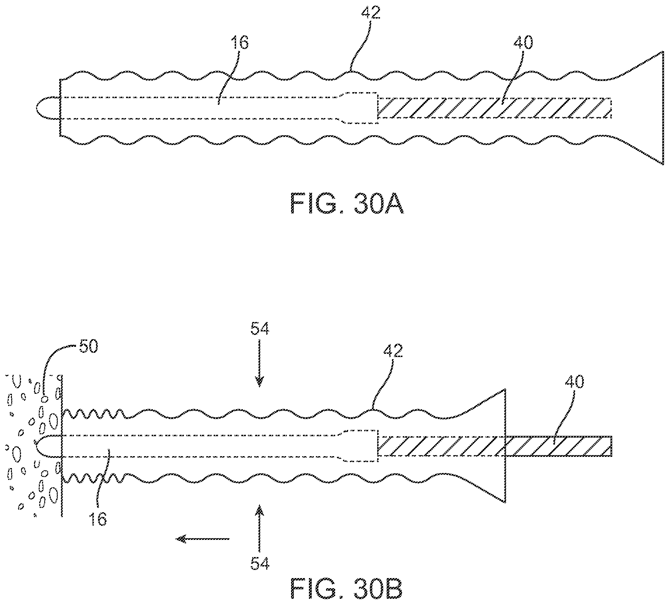

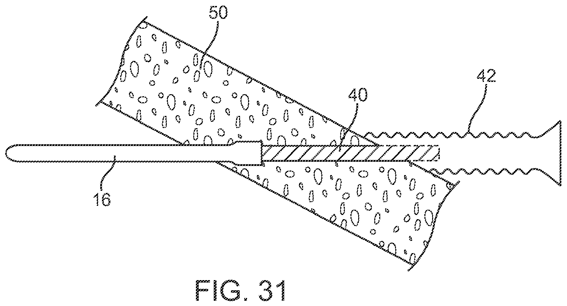

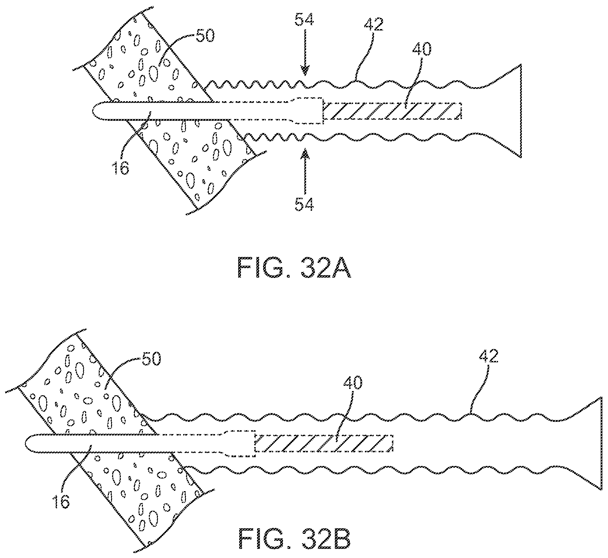

In certain embodiments, the sheath 42 is a self-buckling sheath such that when the needle 10 is inserted into the dermis 50, the sheath 42 buckles or cripples against the skin (Fig. 32A and Fig. 30). As shown in Fig. 30B, as long as there is some un-buckled sheath distal to the grab point 54 (i.e., between the skin and the point at which the clinician grasps the needle), the clinician can continue to insert the needle 10 into the dermis. However, once the sheath is fully compressed distal to the grab point 54, the sheath 42 must be pulled back. In some embodiments, once buckled and the tension at the grab point is removed, the sheath 42 remains buckled (Fig. 31). In another embodiment, once buckled and the tension at the grab point is removed, the sheath 42 self-elongates (Fig. 32A and Fig. 32B).

The following items are also part of the invention

### Items:

1. A needle for delivering a dermal filler comprising:
   a tubular body having a proximal portion and a distal portion,
   a coupler in its proximal portion for attaching a dermal filler thread to the needle, and
   a blunt distal end in the distal portion.
2. The needle of item 1, wherein the blunt distal end does not have an angle that is smaller than 90 degrees.
3. The needle of item 1 or 2, wherein the blunt distal end comprises a tip portion having a height less than diameter of the base.
4. The needle of item 1, wherein the coupler in its proximal portion mechanically attaches a dermal filler thread to the needle.
5. The needle of any preceding item, wherein the coupler comprises at least one slit along the same axis of the tubular body.
6. The needle of item 5, wherein the coupler comprises a plurality of slits.
7. The needle of any preceding item, wherein the coupler comprises at least one strut.
8. The needle of item 7, wherein the coupler comprises a plurality of struts.
9. The needle of item 8, wherein the coupler comprises an even number of struts and each strut of the pair is located along the same longitudinal plane.
10. The needle of item 8, wherein the struts are staggered along the length of the coupler.
11. The needle of any preceding item, wherein the coupler comprises at least one cleat.
12. The needle of item 11, wherein the cleat is tab-shaped or tooth-shaped.
13. The needle of item 1, wherein the coupler comprises a funnel having a proximal and a distal end.
14. The needle of item 1, wherein the coupler is a hypotube coupler.
15. The needle of item 1, wherein the coupler is a detachable coupler.
16. A kit comprising a needle of any preceding item, and a thread optionally coupled thereto which is biocompatible and compressible.
17. The kit of item 16, wherein the thread comprises hyaluronic acid, salt, hydrate or solvate thereof optionally wherein at least a portion of the hyaluronic acid, salt, hydrate or solvate thereof is cross-linked.
18. The kit of item 16 or 17, further comprising a sheath.
19. The kit of item 18, wherein the sheath comprises a pull tab.
20. The kit of item 17 or 19, wherein the sheath is a self-buckling sheath.
21. The kit of any one of items 16 to 20, wherein the thread is coupled to the needle.
22. The kit of item 21, wherein the thread is mechanically coupled to the needle.

## Claims

1. A kit, comprising:
a dermal filler thread; and
a needle (10) for delivering a dermal filler comprising:
a tubular body (16) having a proximal portion (14, 20) and a distal portion,
a coupler (18) in its proximal portion for attaching the dermal filler thread (40) to the needle, and
a blunt distal end (12) in the distal portion.

2. Kit of claim 1,
wherein the coupler (18) is a separate, hollow, tubular piece.

3. Kit of any preceding claim,
wherein the coupler (18) comprises at least one slit (22) along an axis of the tubular body.

4. Kit of any preceding claim,
wherein the coupler comprises at least one strut (24).

5. Kit of any preceding claim,
wherein the coupler comprises at least one cleat (30).

6. The kit of any preceding claim, wherein the blunt distal end (12) does not have an angle that is smaller than 90 degrees and/or wherein the blunt distal end (12) comprises a tip portion (34) having a height less than a diameter of the base.

7. The kit of any preceding claim, wherein the coupler (18) in its proximal portion mechanically attaches a dermal filler thread (40) to the needle.

8. The kit of claim 3 or 4, wherein the coupler (18) comprises a plurality of slits along the same axis of the tubular body.

9. The kit of any of the preceding claims, wherein either the coupler comprises an even number of struts and each strut of the pair is located along the same longitudinal plane, or wherein the struts are staggered along the length of the coupler.

10. The kit of claim 5, wherein the cleat (30) is tab-shaped or tooth-shaped.

11. The kit of claim 1 or 2, wherein the coupler comprises a funnel having a proximal (20) and a distal end (18).

12. The kit of claim 1 or 2, wherein the coupler is a hypotube coupler.

13. The kit of any preceding claim, wherein the dermal filler thread is biocompatible and compressible and/or wherein the dermal filler thread comprises hyaluronic acid, salt, hydrate or solvate thereof, optionally wherein at least a portion of the hyaluronic acid, salt, hydrate or solvate thereof is cross-linked with butanediol diglycidyl ether (BDDE), divinyl sulfone (DVS) or 1-ethyl-3-(3-dimethylaminopropyl) carbodiimide hydrochloride (EDC).

14. The kit of any preceding claim, further comprising a sheath (42), the sheath preferably comprising a pull tab, and/or wherein the sheath is a self-buckling sheath.

15. The kit of any preceding claim, wherein the dermal filler thread is coupled to the needle, and, optionally wherein the dermal filler thread is mechanically coupled to the needle.
